# EUROPEAN PATENT APPLICATION

(11) **EP 1 548 128 A1**
(43) Date of publication of application: **29.06.2005**
(21) Application number: 03798488.7
(22) Date of filing: 25.09.2003
(51) Int. Cl.: C12Q 1/68, C12N 15/09

(54) **METHOD OF RECOVERING DNA**

(30) Priority: 26.09.2002 JP 2002280660
(71) Applicant: Aisin Seiki Kabushiki Kaisha, Kariya-shi, Aichi, 448-8650 (JP)
(72) Inventor: SUZUKI, Katsuhisa, Kisarazu-shi, Chiba 292-0043 (JP)
(74) Representative: TBK-Patent
(86) International application number: PCT/JP2003/012214
(87) International publication number: WO 2004/029292

(57) **Abstract**

It is intended to provide a method for DNA recovery capable of more reliably recovering a cDNA fragment of interest from a group of cDNA fragments in which various kinds of cDNA fragments are present. The method for DNA recovery comprises a first PCR step for carrying out a PCR reaction on the group of cDNA fragments each having a first adaptor sequence on one end and a second adaptor sequence on the other end, using a first primer having a sequence complementary to the first adaptor sequence and also having a labeling substance and a second primer having a sequence complementary to the second adaptor sequence. Also, an electrophoretic step for carrying out a gel electrophoresis on the group of cDNA fragments amplified in the first PCR step and, on the basis of a result of the electrophoretic step, a recovery step for cutting out a gel portion containing a cDNA fragment of interest from a gel and then recovering the cDNA fragment from the gel are comprised.

## Description

### [Technical Field]

The present invention relates to a method for recovering a DNA fragment of interest, and more specifically, the present invention relates to a DNA recovery method for recovering a cDNA fragment of interest from a group of cDNA fragments in which various kinds of cDNA fragments are present.

### [Background Art]

Heretofore, several methods for recovering DNA, in which a cDNA fragment of interest is recovered from a group of cDNA fragments in which various cDNA fragments are present, have been known in the art.

For instance, there is a method that uses a slab gel to recover a cDNA fragment of interest from a group of cDNA fragments (see, e.g., Non-Patent Document 1). That is, for the group of cDNA fragments, for example, an acrylamide gel electrophoresis is carried out. Subsequently, cDNA fragments in acrylamide gel are stained with ethidium bromide and the acrylamide gel is then illuminated with a UV transilluminator to detect cDNA fragments. Subsequently, the acrylamide gel portion containing the cDNA fragment of interest is cut out of the group of fractionated cDNA fragments, followed by recovering the cDNA fragment from the gel.

Furthermore, for instance, there is another method that uses a capillary electrophoresis to recover a cDNA fragment of interest from a group of cDNA fragments (see, e.g., Patent Documents 1 to 3). That is, the group of cDNA fragments previously labeled with a fluorescent probe is provided to a capillary column to carry out electrophoresis. Then, on the basis of a detected peak, a time at which the cDNA of interest will be electrophoresed is estimated. Next, the electrophoresis is performed again using the capillary column, followed by recovering the cDNA of interest on the basis of the estimated time thereof.
[Non-Patent Document 1] A new method for sequencing DNA, Proc. Natl. Acad. Sci., 1977, 74: 560-564
[Patent Document 1] Japanese unexamined patent publication No. 2000-88803
[Patent Document 2] Japanese unexamined patent publication No. 7-181164
[Patent Document 3] Japanese unexamined patent publication No. 6-138037

However, the former method has a problem in that the cDNA fragment of interest cannot be recovered when the content of the cDNA fragment of interest is small because the cDNA fragment cannot be detected under a UV transilluminator even if the cDNA fragment is stained after gel electrophoresis.

On the other hand, in the latter method, because of using cDNA fragments labeled with a fluorescent probe, it is possible to detect the cDNA fragment of interest even if the amount of the cDNA fragment of interest is small. However, in this method, the cDNA fragment cannot be recovered while visually observing. Thus, the cDNA fragment of interest is recovered by predicting a time at which the cDNA of interest will appear on the tip portion of the capillary column. Therefore, the recovered cDNA fragments may be a mixture of the cDNA of interest and other cDNA fragments having lengths which are substantially the same as that of the cDNA fragment. Compared with the case using a slab gel, the method has lower resolution of size fractionation, so that the recovered cDNA fragments may be a mixture including fragments other than that of interest or a recovery rate of the cDNA of interest may be decreased.

The present invention has been made in consideration of the present circumstances and an object of the present invention is to provide a method for DNA recovery, in which a cDNA fragment of interest can be recovered more reliably from a group of cDNA fragments in which various kinds of cDNA fragments are present.

### [Disclosure of the Invention]

Means for attaining the above-mentioned object is a method for DNA recovery, characterized by comprising: a first PCR step for carrying out a PCR reaction on a group of cDNA fragments each having a first adaptor sequence on one end and a second adaptor sequence on the other end, using a first primer having a sequence complementary to the above-mentioned first adaptor sequence and also having a labeling substance and a second primer having a sequence complementary to the above-mentioned second adaptor sequence; an electrophoretic step for carrying out a gel electrophoresis on the group of cDNA fragments amplified in the above-mentioned first PCR step; and, on the basis of the results of the electrophoretic step, a recovery step for cutting out a gel portion containing a cDNA fragment of interest from the gel and then recovering the cDNA fragment from the gel portion.

The DNA recovery method of the present invention is a method for recovering a cDNA fragment of interest from a group of cDNA fragments each having adaptor sequences on both ends. At first, using a fist primer having a sequence complementary to a first adaptor on one end of the cDNA fragment and also having a labeling substance and a second primer having a sequence complementary to a second adaptor sequence on the other end of the cDNA fragment, a PCR reaction is carried out. Then, the amplified group of cDNA fragments (a PCR product) is subjected to a gel electrophoresis. Subsequently, on the basis of the results of the electrophoresis, a gel portion containing the cDNA fragment of interest is cut out of the gel, from which the cDNA fragment is recovered.

According to such a method, the PCR reaction is carried out in advance of the gel electrophoresis and thus a large amount of the group of cDNA fragments can be amplified. Therefore, even if the cDNA fragment of interest is present at a low concentration, it can be amplified to a large extent and can be detected by the gel electrophoresis.

Additionally, in this PCR reaction, a primer having a labeling substance is used, so that the PCR product will also have the labeling substance. Therefore, even if the cDNA fragment of interest remains in a comparatively small amount even after carrying out the PCR reaction, recognizing the labeling substance in the electrophoresis allows the position of the cDNA fragment of interest in a gel to be easily detected.

Furthermore, the group of cDNA fragments is fractionated by gel electrophoresis. Compared to electrophoresis with a capillary column, therefore, an improvement in size resolution is attained and thus only the cDNA of interest can be specifically recovered.

Here, the group of cDNA fragments, which consists of various cDNA fragments provided as a sample may be any of those each having a first adaptor sequence on one end and a second adaptor sequence on the other end. That is, the cDNA fragments may originate from any source, including those prepared from mRNA extracted from animal or plant cells or those prepared from mRNA extracted from viruses or microorganisms. In addition, the group of cDNA fragments may be one that contains almost all genes expressed in cells used in the extraction or one that contains a part of genes expressed therein. Also, the cDNA fragment may be one having adaptor sequences on both ends of the whole-length cDNA or one having adapter sequences on both ends of a fragment of the whole-length cDNA. Besides, each of the first adaptor sequence and the second adaptor sequence may be one having any given sequences, but preferably those designed in consideration of the efficiency of PCR or the like. That is, when each of these adaptor sequences has approximately 15 bases, a stable PCR reaction can be carried out to efficiently amplify the cDNA fragments.

The first primer may be any one having a sequence complementary to the first adaptor sequence and the labeling substance. The complementary sequence as described herein is not limited to a sequence with 100% complementarity to the first adaptor sequence but includes a sequence which is substantially complementary to the first adaptor sequence as far as the cDNA fragments may be amplified in the PCR reaction. In addition, the first primer is not limited to the one that consists of only a sequence complementary to the first adaptor sequence but includes any one in which another sequence is connected to a sequence complementary to the first adaptor sequence. The first primer may be not only the one that corresponds to the entire first adaptor sequence but also any one that corresponds to a part of the first adaptor sequence.

Furthermore, the labeling substance may be any one having high detection sensitivity in gel electrophoresis. Specific examples of fluorescent substances include 6-carboxy fluorescein (hereinafter, referred to as FAM), 4,7,2',4',5',7'-hexachloro-6-carboxy fluorescein (hereinafter, referred to as HEX), NED (Applied Biosystems Japan, Co., Ltd.), 6-carboxy-X-rhodamine (hereinafter, referred to as Rox) and the like. These labeling substances are to be bonded to, for example, an end of the primer DNA (e.g., 5'-end thereof).

The second primer may be any one having a sequence complementary to the second adaptor sequence. The complementary sequence described herein is not limited to a sequence with 100% complementarity to the second adaptor sequence but includes a sequence which is substantially complementary to the second adaptor sequence as far as the cDNA fragments may be amplified in the PCR reaction. In addition, the second primer is not limited to the one that consists of only a sequence complementary to the second adaptor sequence but includes also any one in which another sequence is connected to a sequence complementary to the second adaptor sequence. In addition, the second primer is not only the one that corresponds to the entire second adaptor sequence but also any one that corresponds to a part of the second adaptor sequence. Furthermore, in addition to the first primer, the second primer may also be bonded to the above-mentioned labeling substance.

Next, various other chemicals generally used in the PCR reaction will be described. Preferable DNA polymerases include those that do not result in permanent inactivation even by short-time heating at high temperatures at the time of producing denatured DNA strands in a PCR reaction and yet show their activities at high temperatures. Examples of the DNA polymerases include those originate from thermophilic bacteria such as Thermococcus litoralis, Bacillus stearothermophilus, Methanothermus fervidus, Thermus aquaticus, T. flavus, T. lacteus, T. rubens, and T. ruber; and those originate from thermophilic archaebacteria such as Desulfurococcus mobilis, Methanobacterium thermoautotrophilcum, Sulfolobus solfataricus, S. acidocaldarius, Thermoplasma acidophilum, and Pyrococcus kodakaraensis strain KOD1. Among them, because of ready availability and so on, the DNA polymerase (Taq DNA polymerase) from Thermus aquaticus, the DNA polymerase from Thermococcus litoralis, or the DNA polymerase from Pyrocuccus kodakaraensis strain KOD1 is preferably employed.

Furthermore, in a PCR reaction solution, for inhibiting the activity of DNA polymerase before the application of nucleic acids, an antibody specific to the DNA polymerase may be mixed. Examples of the antibody include a monoclonal antibody, a polyclonal antibody, a recombinantly-engineered antibody, and a chemically- or recombinantly-engineered antibody fragment (e.g., Fab fragment). Among them, the monoclonal antibody is particularly preferably used. For instance, a known monoclonal antibody against Taq DNA polymerase is able to inhibit the enzyme activity of Taq DNA polymerase at temperatures of about 20°C to about 40°C, while it is inactivated at high temperatures in a thermal cycle of the PCR.

Furthermore, in general, the PCR cycle is carried out in the presence of four different dNTPs, i.e., dATP, dCTP, dGTP, and dTTP.

In addition, generally, the PCR reaction is carried out in a reaction solution that contains an appropriate buffering agent. This is for efficient amplification of nucleic acids. Depending on the DNA polymerase or the like, the buffer solution can be appropriately changed or modified to obtain optimal reaction conditions. For example, a buffer solution prepared by adding potassium chloride or magnesium chloride to a Tris-based buffer solution with an appropriately-adjusted pH value can be used.

Furthermore, in the PCR reaction solution, 5%-10% DMSO and 1%-2% betaine may be added. It has an effect of keeping a problem at minimum, wherein a product is hardly amplified when a cDNA fragment to be provided as a template DNA has a secondary structure.

In the electrophoresis step, by using a known slab electrophoresis or the like, such as an acrylamide gel electrophoresis or an agarose gel electrophoresis, or the like, cDNA fragments (PCR products) are electrophoresed to fractionate the cDNA fragments. For the electrophoresis, any of electrophoretic devices known in the art may be employed.

The recovery step may be, on the basis of the results of the electrophoresis step, any one using any of procedures as far as it is a step of recovering the cDNA fragment of interest by cutting a gel portion containing the DNA fragment out of the gel.

In other words, for example, as will be illustrated in examples described later, there is a method wherein the electrophoresis step is carried out by subjecting cDNA fragments to a DNA sequencer capable of detecting a labeling substance and, from the results of analysis, a cDNA fragment to be recovered is determined. Subsequently, the same sample is electrophoresed again and, when the cDNA fragment of interest is detected, the gel portion containing the cDNA fragment of interest is then cut out, followed by recovering the cDNA fragment therefrom.

In addition, there is a method wherein, after gel electrophoresis, a gel is placed on a scanner capable of detecting a labeling substance and, from the results of analysis, the cDNA fragment to be recovered is determined. Subsequently, the gel portion containing the cDNA fragment of interest is cut out, followed by recovering the cDNA fragment therefrom.

Now, the group of cDNA fragments each having adaptor sequences on both ends can be prepared, for example, as follows:

That is, a method can be used to produce a group of cDNA fragments each having adaptor sequences on both ends, wherein the method comprises the steps of: synthesizing a cDNA fragment added with a tag substance on the 5' end thereof from mRNA which is extracted from a cell; cleaving the obtained cDNA fragment with a first restriction enzyme; recovering a cDNA fragment having the tag substance by binding to a high-affinity substance having high affinity to the above-mentioned tag substance; binding the recovered cDNA fragment with a first adaptor sequence having a sequence complementary to a sequence of an enzyme-cleaving site cleaved by the first restriction enzyme; cleaving the cDNA fragment bonded to the above-mentioned first adaptor sequence by a second restriction enzyme; recovering a cDNA fragment free of the above-mentioned tag substance by biding to the above-mentioned high-affinity substance to remove the cDNA fragment having the above-mentioned tag substance; and biding the cDNA fragment free of the above-mentioned tag substance to a second adaptor sequence having a sequence complementary to a sequence of an enzyme-cleaving site cleaved by the above-mentioned second restriction enzyme.

Furthermore, regarding such a group of cDNA fragments, it is also possible to divide the group of the cDNA fragments into a group of small-sized cDNA fragments by using a step, wherein the step comprises a PCR reaction using a primer having a sequence complementary to the above-mentioned first adaptor sequence and having an arbitrary two-base sequence NN on the 3' end of the sequence and a primer having a sequence complementary to the above-mentioned second adaptor sequence and having an arbitrary two-base sequence NN on the 3' end of the sequence.

By preparing a group of cDNA fragments using such a method, it is possible to contain almost all genes which has been expressed, i.e., known genes and unknown genes alike in that group. Therefore, this can be used effectively in gene analysis and so on. Besides, in order to make the analysis easier, the group of cDNA fragments can be divided into a plurality of small-sized groups of cDNA fragments.

Furthermore, the tag substance and the high-affinity substance having high affinity to the tag substance refer to substances that constitute a biding pair capable of specifically biding to each other with high affinity. As a combination of the tag substance and the high-affinity substance, for example, biotin and streptoavidine, biotin and avidin, FIGT and FITC antibody, DIG and anti-DIG, Protein A and mouse IgG, latex particles, and so on can be cited. In addition, in each of the combinations, any of them may be used as a tag substance or any of them may be used as a high-affinity substance.

Furthermore, a restriction enzyme refers to an enzyme, also called a restriction endonuclease in general, which hydrolyzes and cleaves double-stranded DNA at a specific sequence. In the above-mentioned method, in order to obtain appropriate fragments, two different restriction enzymes (first restriction enzyme and second restriction enzyme) are used in combination. The restriction enzymes used are preferably those capable of cutting a cDNA fragment into fragments of recognizable lengths. Also the enzymes are preferably those which can cleave more of synthesized cDNA fragments, preferably almost all of them. Besides, as a restriction enzyme, a four-base recognition enzyme or a six-base recognition enzyme may be used. In particular, from the reason described above, the use of the four-base recognition enzyme is preferable.

Furthermore, the adaptor sequence, which is used for binding to a primer used in PCR amplification, is designed on the basis of the restriction enzyme used. That is, the first adaptor sequence for binding to the enzyme-cleaving site of the first restriction enzyme has a sequence complementary to the enzyme-cleaving site of the first restriction enzyme. In addition, the second adaptor sequence for binding to the enzyme-cleaving site of the second restriction enzyme has a sequence complementary to the enzyme-cleaving site of the second restriction enzyme.

Furthermore, the primer set consists of a set of primers used to amplify cDNA fragments by means of PCR. The "arbitrary two-base sequence NN" used herein refers to a sequence arbitrarily selected from A, T, G, and C. Each given sequence comprises two bases as a result of considering simplicity and analytical accuracy of the method. In other words, by making each given sequence from two bases, it is possible to prepare a maximum of 256 different primer sets, so that the group of cDNA fragments can be divided into a plurality of groups. In addition, the given two-base sequence NN may be provided as one having three or more bases for one of the primers or for both of the primers. By this procedure, it is possible to increase the number of the kinds of primers, so that the number of primer sets can be made to a maximum of 1,024 or a maximum of 4,096.

Furthermore, the method for DNA recovery according to any of those described above may be preferably provided as a method for DNA recovery characterized by comprising a second PCR step for carrying out again a PCR reaction on the cDNA fragment recovered in the above-mentioned recovery step, using a third primer having a sequence complementary to the above-mentioned first adaptor sequence and a fourth primer having a sequence complementary to the above-mentioned second adaptor sequence.

In the present invention, for the cDNA fragment recovered in the recovery step, a second PCR step is provided for carrying out the PCR reaction again. Therefore, even if the amount of the cDNA fragment of interest is small, it can be amplified substantially.

In addition, the third primer used in this PCR step may be any one having a sequence complementary to the first adaptor sequence. For example, the above-mentioned first primer may be used. In this PCR step, however, a labeling substance is not particularly required. Likewise, the fourth primer may be any one having a sequence complementary to the second adaptor sequence. For example, the above-mentioned second primer may be used.

Furthermore, the method for DNA recovery according to the method described above may be preferably provided as a method for DNA recovery characterized by performing the second PCR step by mixing, in the second PCR step, a homologous recombinant protein containing at least either of a RecA protein or a RecA-altered protein obtained by altering the RecA protein and having functions analogous to those of the RecA protein.

According to the present invention, in the second PCR step, the homologous recombinant protein such as the RecA protein or the like is mixed in the reaction solution and then PCR is carried out, followed by amplifying recovered cDNA fragments.

By carrying out the PCR in this way, it is possible to prevent a decrease in the yield of the cDNA fragment, while keeping the amplification of by-products (nonspecific PCR products) suppressed. That is, the presence of the above homologous recombinant protein prevents a primer extension reaction due to binding of the primer to a nonspecific region of the cDNA fragment, so that the amplification of nonspecific PCR products can be prevented.

In addition, in the present invention, as the specificity is high as described above, even if the temperature conditions of the primer extension reaction, such as an annealing temperature, are changed, the recovered cDNA fragments can be specifically amplified. That is, in the conventional PCR method, when the temperature of the primer extension reaction, such as the annealing temperature, is set to be low, the recovered cDNA fragments are amplified together with the amplification of a large amount of by-products. According to the present invention, however, it becomes possible to amplify the recovered cDNA fragments more specifically.

From these results, the cDNA of interest can be recovered more reliably.

Here, the homologous recombinant protein described above may be any one containing at least a RecA protein or a RecA-altered protein obtained by altering the RecA protein and having functions analogous to those of the RecA protein. Examples of the RecA-altered protein include a product of a gene produced from a gene that encodes the RecA protein by the induction of a site-specific mutation or the like and comprises an amino acid sequence having one or several amino acids being deleted, substituted, or added, while having functions analogous to those of the RecA protein. Alternatively, it may be a protein fragment of the RecA protein (RecA fragment) having functions analogous to those of the protein.

Furthermore, the RecA protein or the like, as in the case with the DNA polymerase described above, is preferably one that does not result in permanent inactivation even when subjected to short-time heating at high temperatures at the time of producing denatured DNA strands in a PCR reaction and yet shows activity at high temperatures. For instance, because of ease of availability etc., a RecA protein originating from E. coli is preferably used.

In addition, the above homologous recombinant protein is preferably mixed in the range of 1 ng to 1,000 ng with respect to 20 pmol of the primer. This is because, when the PCR is carried out in such a range, the cDNA fragment can be efficiently and specifically amplified.

Furthermore, the method for DNA recovery according to any of the methods for DNA recovery described above may be provided as a method for DNA recovery characterized by comprising: a ligation step in which, after the recovery step when there is no second PCR step, or after the second PCR step when there is the second PCR step, the above-mentioned cDNA fragment is ligated to a plasmid vector to form a recombinant plasmid; and an introduction step for introducing the above-mentioned recombinant plasmid into E. coli.

The method for DNA recovery of the present invention comprises, after the recovery step if there is no second PCR step, or after the second PCR step if there is a second PCR step, a ligation step in which a cDNA fragment of interest is ligated to a plasmid vector to form a recombinant plasmid. Furthermore, the method further comprises an introduction step for introducing the recombinant plasmid into E. coli.

In this way, by ligating the recovered cDNA fragment to the plasmid vector and then introducing the same into E. coli, it is useful in the structural analysis for the determination of base sequence or the like. That is, when the transformed E. coli is incubated and a plasmid DNA having the cDNA fragment is extracted from the transformed E. coli, the plasmid DNA can be used in a structural analysis for the determination of the base sequence.

### [Brief Description of Drawings]

Fig. 1 is, for Example 1, an explanation diagram that illustrates the outline of a method of preparing a group of cDNA fragments to be used as a sample for the recovery of a cDNA fragment of interest.
Fig. 2 is, for Example 1, an explanation diagram that illustrates the details of the method of preparing the group of cDNA fragments to be used as the sample for the recovery of the cDNA fragment of interest.
Fig. 3 is a diagram for Example 1, where A is an explanation diagram illustrating a first adaptor sequence and B is an explanation diagram illustrating a second adaptor sequence.
Fig. 4 is, for Example 1, an explanation diagram that illustrates a sequence of a primer set for dividing into 256 different groups of cDNA fragments.
Fig. 5 is, for Example 1, an explanation diagram that illustrates a method of recovering the cDNA fragment of interest from the group of cDNA fragments.
Fig. 6 is, for Example 3, a photograph substituted for the drawing and illustrating the results of electrophoresis performed on the group of cDNA fragments amplified by RT-PCR.

### [Best Mode for Carrying Out the Invention]

Hereinafter, examples of the present invention will be described with reference to the drawings.

### [Example 1]

In the present example, at first, genes expressed in certain cells are grouped as described below. First of all, a group of cDNAs is synthesized from extracted mRNAs. Then, cDNAs of the obtained group are cleaved by two appropriate restriction enzymes, and both cleaved ends thereof are connected with adaptor sequences to prepare a group of cDNA fragments, each having a length enough to be recognized and having the adaptor sequences at both ends. Subsequently, the obtained group of cDNA fragments is divided into a plurality of groups by using a plurality of different primer sets.

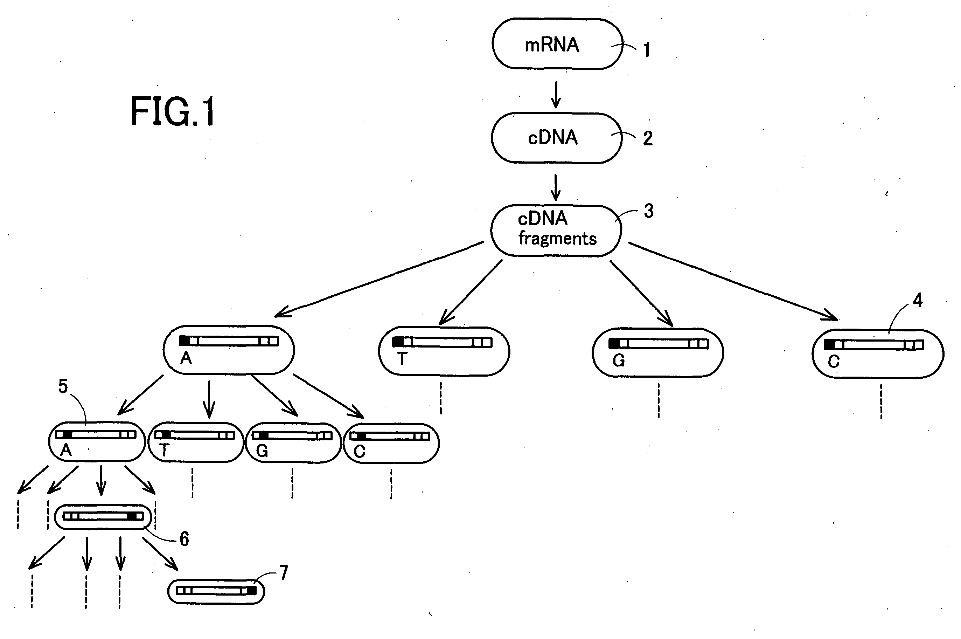

Referring now to Fig. 1, the classification method will be described. From Group 1 consisting of the expressed mRNAs, Group 2 of cDNAs is synthesized by a method known in the art. Then, Group 2 is subjected to cleavage with two appropriate restriction enzymes to obtain Group 3 of cDNA fragments. Subsequently, Group 3 of cDNA fragments is further divided on the basis of the sequence for two bases each at both ends of the cDNA, i.e., the sequences for four bases in total, that is, depending on whether the four bases are A, T, G, or C. For instance, by differentiating the base at the 5' end (represented by a black-colored portion in Fig. 1), it is possible to divide Group 3 into four different kinds of Group 4. Then, by differentiating the subsequent second base at the 3' end (represented by a black-colored portion in Fig. 1), it is possible to divide Group 4 into four different kinds of Group 5. Then, by differentiating the subsequent second base at the 3' end (represented by a black-colored portion in Fig. 1), it is possible to divide Group 5 into four different kinds of Group 6. Furthermore, by differentiating the base at the 3' end (represented by a black-colored portion in Fig. 1), it is possible to divide Group 6 into four different kinds of Group 7.

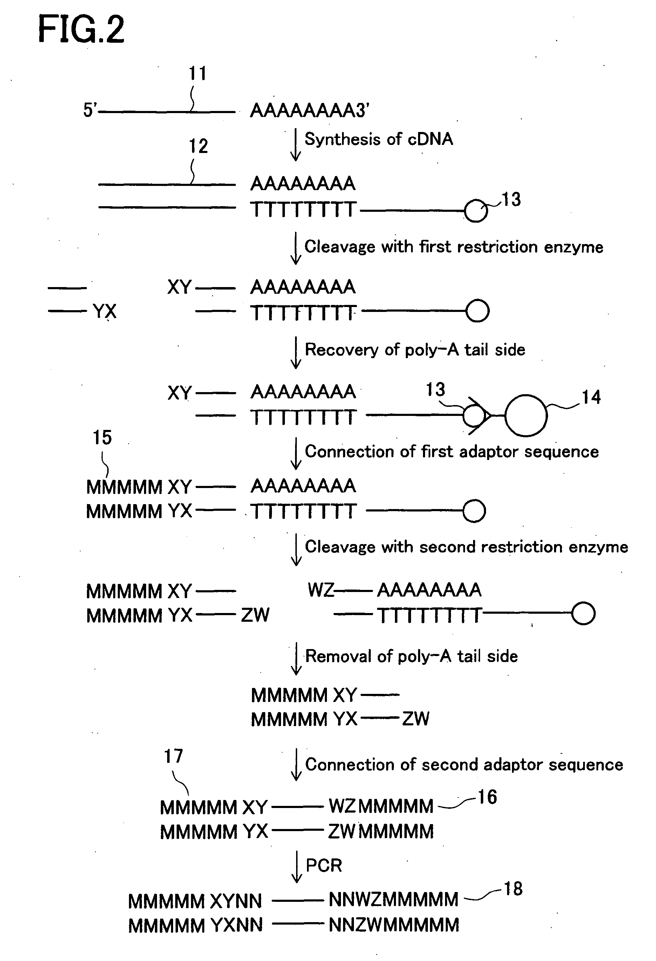

Next, a method for preparing a plurality of Groups 7 of cDNA fragments from Group 1 of extracted mRNAs will be specifically described with reference to Fig. 2. Although each letter of alphabets in Fig. 2 indicates a base that constitutes a base sequence, N, M, W, X, Y and Z each represents an arbitrary base, wherein X and Y bind complementarily to each other and W and Z bind complementarily to each other.

First, mRNA 11 is extracted from a certain cell to be tested by a method known in the art. In the present example, using a Fast Track 2.0 kit (manufactured by Invitrogen Corporation), 20 µg in total of mRNA 11 was extracted from yeast.

Next, an oligo-dT primer complementary to a poly-A tail at the 3' end of the extracted mRNA 11 is labeled with biotin (tag substance) 13. This is used as a primer to synthesize cDNA 12. Specifically, 20 µg of the extracted mRNA 11 was mixed with 100 pmole in 0.8 µl of a 5'-biotinylated oligo-dT primer (manufactured by BRL, Inc.), followed by incubation at 65°C for 5 minutes. After cooling on ice, it was incubated in 20.0 µl of a reverse transcription buffer, with 5 mM of MgCl₂, 0.5 mM of dNTP Mix (manufactured by BRL, Inc.), and 10 mM of DTT (manufactured by BRL, Inc.) in final concentrations at 42°C for 60 minutes. Thereafter, incubation was performed in 150.0 µl of a double strand synthesis buffer, with 0.27 mM of dNTP Mix (manufactured by BRL, Inc.), 1.33 mM of DTT (manufactured by BRL, Inc.), 20.0 units of E. coli ligase (manufactured by BRL, Inc.), 40.0 units of E. coli DNA polymerase (manufactured by BRL, Inc.), and 2.0 units of RNase H (manufactured by BRL, Inc.) in final concentrations at 16°C for 120 minutes, followed by incubation at 70°C for 15 minutes to terminate the reaction.

Then, the synthesized cDNA 12 is cleaved with a first restriction enzyme. Specifically, 20 units of a restriction enzyme MspI (manufactured by Takara Shuzo Co., Ltd.) and 10 µg of the cDNA 12 in final concentrations in 100 µl were reacted at 37°C for 360 minutes. Incidentally, MspI is a restriction enzyme that recognizes four bases. Subsequently, the cDNA fragments were purified with ethanol by a method known in the art.

Following that, using streptavidin (a high affinity substance) 14, the biotin 13 is captured and only 3'-end fragments of the cleaved cDNA fragments are recovered. Specifically, the biotin 13 was connected to the streptavidin 14 (manufactured by Dynal, Inc.) immobilized on magnetic beads in a reaction solution to obtain the product.

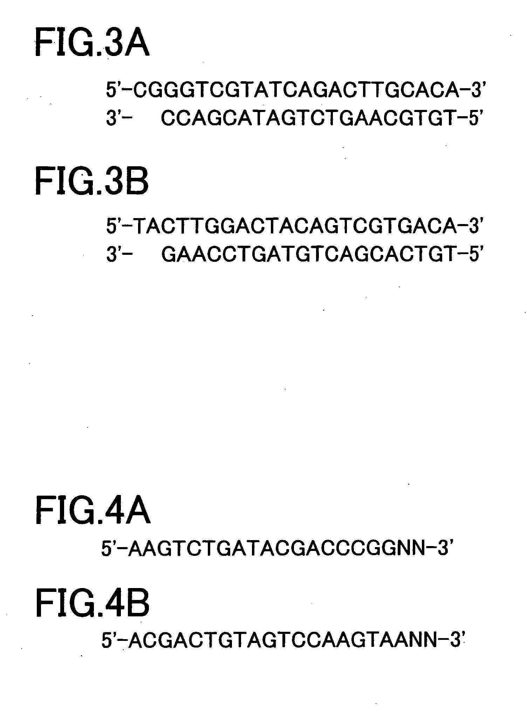

Then, a first adaptor sequence 15 having a sequence complementary to the recognition cleavage site of the first restriction enzyme is ligated to the 5' end of the recovered cDNA fragments. The first adaptor sequence 15 used in the present example is shown in Fig. 3A. Specifically, using 5.0 µg of the first adaptor (manufactured by BRL, Inc.) having a CG protruding end (i.e., a sequence complementary to the cleavage fragment site of the restriction enzyme MspI) and 10 units of T4 DNA ligase (manufactured by NEB, Inc.), ligation was carried out in 15 µl of a T4 DNA ligase buffer.

Next, this cDNA fragment is cleaved with a second restriction enzyme. Specifically, a restriction enzyme MseI (manufactured by NEB, Inc.) with a final concentration of 50 units in 200 µl and the cDNA fragment were reacted at 37°C for 360 minutes. Incidentally, this MseI is also a restriction enzyme that recognizes four bases. Following that, cDNA fragments were purified with ethanol by a method known in the art.

Then, biotin 13 was captured using streptavidin 14 and a 3'-end fragment of cleaved cDNA fragments is removed while a 5'-end fragment of cleaved cDNA fragments is recovered.

Furthermore, to the 3' end of the recovered cDNA fragment, a second adaptor sequence 16 having a sequence complementary to the recognition cleavage site of the second restriction enzyme is attached. The second adaptor sequence 16 used in the present example is shown in Fig. 3B. Specifically, using 10 pmole of the second adaptor sequence (manufactured by BRL, Inc.) having a TA protruding end (i.e., a sequence complementary to the cleavage fragment site of the restriction enzyme MseI) and 10 units of T4 DNA ligase (manufactured by NEB, Inc.), ligation was carried out in 10 µl of a T4 DNA ligase buffer.

A group of cDNA fragments 17 which contain known sequences at both ends has been constructed by the above procedures.

Next, this group of cDNA fragments 17 is subjected to a PCR reaction using a primer set. This primer set uses: as shown in Fig. 4A, a primer having a sequence complementary to the first adaptor sequence and a primer having an additional sequence of two bases (represented by NN in Fig. 4) in the direction of amplification; and, as shown in Fig. 4B, a primer having a sequence complementary to the second adaptor sequence and an additional sequence of two bases (represented by NN in Fig. 4) in the direction of amplification. Since two bases added to the individual primers in the direction of amplification are designed by all the combinations of the four different bases of A, T, G, and C, a maximum of 256 different primer sets are contemplated. Thus, by performing PCR reaction for the group of cDNA fragments 17 using these primer sets, it is possible to group into a plurality of kinds of group of cDNA fragments 18 and to perform PCR amplification. PCR may be carried out by a method known in the art.

The plurality of kinds of group of cDNA fragments 18 obtained as above each can be utilized as a sample for recovering a cDNA fragment of interest in the present example.

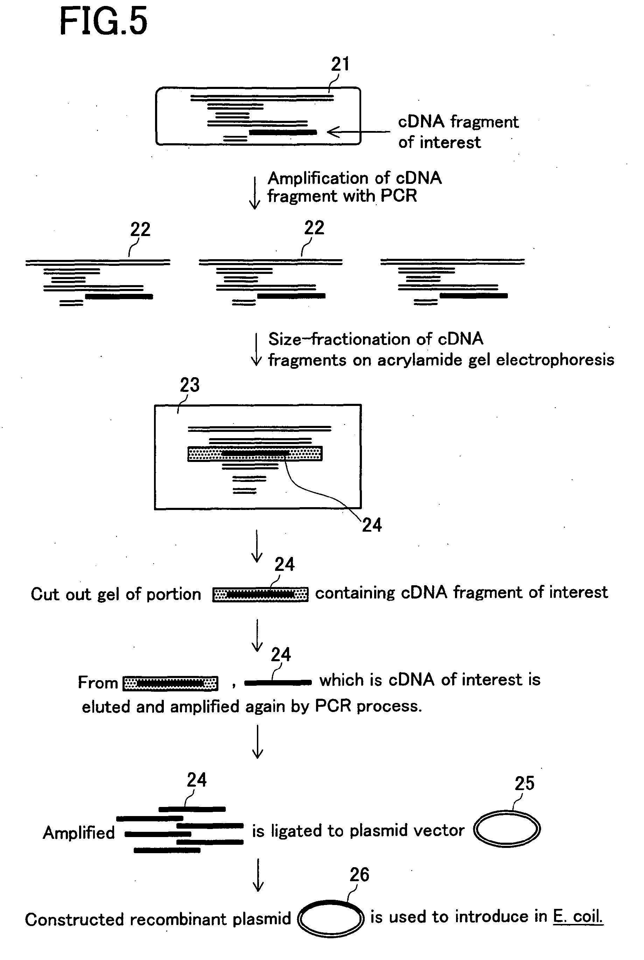

Next, one of small-scale groups of cDNA fragments created as mentioned above is used as a sample to recover the cDNA fragment of interest by a method shown below. This method will be described with reference to Fig. 5.

Additionally, the small-scale group of cDNA fragments as a sample used in the present example is classified by using a primer composed of oligonucleotide 1 and a primer composed of oligonucleotide 2.

Oligonucleotide 1:
5'-aagtctgatacgacccggac-3'

Oligonucleotide 2:
5′-acgactgtagtccaagtaata-3′

First, in a first PCR step, Group 21 of the created cDNA fragments is amplified by PCR reaction using a first primer that has a sequence complementary to the first adaptor sequence 15 and is connected to a labeling substance and a second primer that has a sequence complementary to the second adaptor sequence 16.

Specifically, the above-mentioned oligonucleotide 1 having a sequence complementary to the first adaptor sequence 15 was subjected to a PCR reaction using the first primer attached to near-infrared fluorochrome IRD-800 that is a fluorescent substance and the second primer composed of the above oligonucleotide 2 that has a sequence complementary to the second adaptor sequence 16. The first and second primers may be synthesized by a method known in the art, respectively.

Also, the PCR reaction was carried out using GeneAmp 2400 available from PerkinElmer, Inc. The PCR conditions were based on Stepdown PCR (see Biotechniques, 1996, 20: 478-485). An enzyme (DNA polymerase) used was a KOD Dash enzyme manufactured by Toyobo Co., Ltd. This DNA polymerase was derived from Pyrococcus kodakaraensis (strain KOD1). The composition of the reaction solution was determined according to an accompanying instruction manual.

Next, in an electrophoresis step, Group 22 of the amplified cDNA fragments (PCR product) is subjected to gel electrophoresis.

Specifically, the Group 22 of amplified cDNA fragments was subjected to a DNA sequencer (LIC-4200L (S) -1 manufactured by LI-COR, Inc.) capable of reading a fluorochromes and then to acrylamide gel electrophoresis for size fractionation. The composition of the acrylamide gel 23 was determined according to an accompanying manual of the above DNA sequencer.

Next, in a recovery step, a gel portion containing a cDNA fragment 24 of interest is cut out of the whole gel on the basis of the results of the electrophoresis step to recover the cDNA fragment 24.

Specifically, a cDNA fragment to be extracted was determined based on the analysis result of acrylamide gel electrophoresis. Incidentally, this cDNA fragment 24 is derived from a known gene A and has a length of 202 bp (including both adaptor sequences). Again, acrylamide gel electrophoresis was carried out for the same sample and terminated immediately after the cDNA fragment 24 of interest appeared on the analysis screen to cut out a gel portion on which the laser of the detector was irradiated.

Then, the acrylamide gel portion which had been cut out was reacted in a buffer overnight at 65°C to recover the cDNA fragment 24 from the acrylamide gel. In order to recover this cDNA fragment 24, a kit manufactured by Omega Bio-tek, Inc. (E.Z.N.A. Poly-Gel DNA extraction kit) was used.

Following that, in a second PCR step, the recovered cDNA fragment 24 is again subjected to a PCR reaction using a third primer having a sequence complementary to the first adaptor sequence 15 and a forth primer having a sequence complementary to the second adaptor sequence 16.

Specifically, a PCR reaction was carried out using at the forward side the third primer having a sequence complementary to the first adaptor sequence in addition to a sequence of a restriction enzyme NotI site, and at the reverse side the forth primer having a sequence complementary to the second adaptor sequence in addition to a sequence of a restriction enzyme SpeI site. The third primer is composed of oligonucleotide 3 and the forth primer is composed of oligonucleotide 4.

Oligonucleotide 3:

5'-tagcggccgcaagtctgatacgacccggac-3'

Oligonucleotide 4:

5′-gaactagtaacgactgtagtccaagtaata-3′

Again in this PCR step, a PCR reaction was carried out using GeneAmp 2400 manufactured by PerkinElmer, Co., Ltd. under the conditions of Stepdown PCR (see Biotechniques, 1996, 20: 478-485). An enzyme (DNA polymerase) used was a KOD Dash enzyme available from Toyobo Co. , Ltd. The composition of the reaction solution was determined according to an accompanying instruction manual.

Next, in a ligation step, a product of the cDNA fragment 24 amplified in the second PCR step is ligated to a plasmid vector 25 to form a recombinant plasmid 26.

Specifically, the amplification product of the cDNA fragment 24 was treated with restriction enzymes NotI and SpeI and then ligated to the plasmid vector (pbluescript II) 25 to form the recombinant plasmid 26. This ligation was performed by using the Ligation kit ver.2 manufactured by Takara Shuzo Co., Ltd. according to an accompanying instruction manual thereof.

Thereafter, in an introduction step, the recombinant plasmid 26 is introduced into E. coli.

Specifically, E. coli DH5α was used as competent cells and the recombinant plasmid 26 was introduced into the E. coli by a method known in the art.

By the method as mentioned above, the cDNA fragment 24 of interest can be recovered more reliably from the Group 21 of cDNA fragments.

Then, cDNA fragments derived from the other known genes (gene B, gene C, and gene D) were recovered by the above method, respectively.

For recovering each of cDNA fragments, groups of cDNA fragments each different from the group of cDNA fragments used in recovering the above gene A were prepared as a sample by the method described above.

In addition, in accordance with the difference in the group of cDNA fragments used as a sample, first to forth primers having slightly different base sequences were used.

Specifically, for recovering cDNA fragments derived from the gene B, oligonucleotide 5 as a first primer, oligonucleotide 6 as a second primer, oligonucleotide 7 as a third primer, and oligonucleotide 8 as a forth primer were used.

Similarly, for recovering cDNA fragments derived from the gene C, oligonucleotide 9 as a first primer, oligonucleotide 10 as a second primer, oligonucleotide 11 as a third primer, and oligonucleotide 12 as a forth primer were used.

Moreover, to recover cDNA fragments derived from the gene D, oligonucleotide 13 as a first primer, oligonucleotide 14 as a second primer, oligonucleotide 15 as a third primer, and oligonucleotide 16 as a forth primer were used.

Oligonucleotide 5:
5'-aagtctgatacgacccggtt-3'

Oligonucleotide 6:
5′-acgactgtagtccaagtaagt-3′

Oligonucleotide 7:
5′-tagcggccgcaagtctgatacgacccggtt-3′

Oligonucleotide 8:
5′-gaactagtaacgactgtagtccaagtaagt-3′

Oligonucleotide 9:
5'-aagtctgatacgacccggtc-3'

Oligonucleotide 10:
5'-acgactgtagtccaagtaagg-3'

Oligonucleotide 11:
5'-tagcggccgcaagtctgatacgacccggtc-3'

Oligonucleotide 12:
5'-gaactagtaacgactgtagtccaagtaagg-3'

Oligonucleotide 13:

5'-aagtctgatacgacccggta-3'

Oligonucleotide 14:
5'-acgactgtagtccaagtaacg-3'

Oligonucleotide 15:
5′-tagcggccgcaagtctgatacgacccggta-3′

Oligonucleotide 16:
5'-gaactagtaacgactgtagtccaagtaacg-3'

Next, a recombinant plasmid was extracted from transformed E. coli by a method known in the art. This plasmid extraction was carried out for a plurality of colonies. For each of the extracted recombinant plasmids, a base sequence of the incorporated cDNA fragments was determined. Such gene analysis was carried out on each of the cDNA fragments derived from the gene A through the gene D in a similar manner. The results thereof are shown in Table 1. It is noted that numbers in parenthesis represent the length of each cDNA fragment (including both adaptor sequences).

**[Table 1]**

| | Recovery rate (%) |
|---|---|
| Gene A (202 bp) | 67 |
| Gene B (314 bp) | 57 |
| Gene C (130 bp) | 100 |
| Gene D (209 bp) | 100 |

As is evident from Table 1, in the recovery of the cDNA fragments derived from the gene A, among the recombinant plasmids subjected to the gene analysis, approximately 67% thereof contained the cDNA fragment of interest. Also, in the recovery of the cDNA fragments derived from the gene B, among the recombinant plasmids subjected to the gene analysis, approximately 57% thereof contained the cDNA fragment of interest. Furthermore, in the recovery of the cDNA fragments derived from the gene C, among the recombinant plasmids subjected to the gene analysis, 100% thereof contained the cDNA fragment of interest. Similarly, in the recovery of the cDNA fragments derived from the gene D, among the recombinant plasmids subjected to the gene analysis, 100% thereof contained the cDNA fragment of interest.

From these results, the recovery of the cDNA fragment of interest from the group of cDNA fragments according to the method described in the present example is found to be capable of recovering the cDNA fragment of interest more reliably.

As explained above, according to the method for recovering DNA in the present example, since the PCR reaction is carried out in advance for a group of selected cDNA fragments, the group of cDNA fragments can be amplified in a substantial amount. Therefore, even if the cDNA fragment of interest has a low concentration, it can be amplified substantially and can be detected in gel electrophoresis.

Furthermore, in this PCR reaction, since the primer having a labeling substance is used, a PCR product also contains the labeling substance. Thus, even if the cDNA fragment of interest is small in number, the position of the cDNA fragment of interest in the gel can be easily detected by recognizing this labeling substance in electrophoresis.

In addition, because the group of cDNA fragments is fractionated by gel electrophoresis, it is possible to improve size resolution and to recover only the cDNA fragment of interest more specifically, as compared to, for example, electrophoresis using a capillary column.

Moreover, since each of the first and the second adaptor is provided as a sequence of approximately 15 bases, a PCR reaction can be stably carried out thereon to amplify cDNA fragments efficiently.

Furthermore, as a labeling substance, the near-infrared fluorochrome IRD-800 which is a fluorescent substance, is used. Thus, the detection sensitivity of a cDNA fragment in gel electrophoresis can be improved.

Additionally, in the present example, a second PCR step is provided to carry out additional PCR reaction for a cDNA fragment that has been recovered in the recovery step. Therefore, it is possible to significantly amplify the recovered cDNA fragment even if the content thereof is small.

In addition, the present invention is provided with a ligation step in which a cDNA fragment is ligated to a plasmid vector to form a recombinant plasmid after the second PCR step and an introduction step in which this recombinant plasmid is introduced into E. coli. Therefore, this is effective in the case of conducting analysis on the structure of the cDNA fragment, and so on. That is, by culturing the E. coli which has been transformed and extracting a plasmid DNA having the cDNA fragment, this extracted plasmid DNA can be utilized in structure analysis (e.g., the determination of a base sequence).

### [Example 2]

Next, a second example will be described. In the following, the description regarding the same procedures as those of the above-mentioned Example 1 will be omitted or simplified.

Even though a cDNA fragment of interest was recovered by the above method, some samples resulted in slightly lower recovery rate (see Table 1). This is because the amount of the cDNA fragment of interest itself is small. Accordingly, if only a gel portion where the cDNA fragment of interest is present could be cut out precisely, the contamination with other cDNA fragments could be reduced, leading to an improvement in the recovery rate of the cDNA fragment of interest. Thus, in order to improve the recovery rate of the cDNA fragment of interest, an experiment was carried out using the following method.

Procedures up to a first PCR step were carried out in the same manner as the above-mentioned Example 1.

Then, in an electrophoresis step, acrylamide gel electrophoresis was performed as in the Example 1 described above. Subsequently, instead of performing additional electrophoresis for the same sample, the gel that had been initially subjected to electrophoresis was flaked off the gel plate by using a filter paper. Subsequently, the gel adhering to the filter paper was placed on a scanner capable of detecting a fluorochrome and the whole gel was subjected to imaging. By this procedure, it was possible to recover accurately only the gel portion containing the target cDNA fragment even if this cDNA fragment was at a lower concentration.

After the recovery step, a second PCR step, a ligation step and an introduction step were conducted in the same manner as the above-mentioned Example 1.

In addition, as described in the above Example 1, a recombinant plasmid was extracted from transformed E. coli by a method known in the art to determine a base sequence of the inserted cDNA fragment. Such gene analysis was conducted for a cDNA fragment derived from a gene E in the same way.

For recovering the cDNA fragment derived from the gene E, as a sample, a group of cDNA fragments different from the groups of cDNA fragments used to recover the above genes A to D was prepared by the above-mentioned method.

Since the group of cDNA fragments given as the sample was different, a first primer through a forth primer having slightly different base sequences thereof were utilized.
Specifically, oligonucleotide 17 as a first primer, oligonucleotide 18 as a second primer, oligonucleotide 19 as a third primer, and oligonucleotide 20 as a forth primer were used.

Oligonucleotide 17:

5'-aagtctgatacgacccggta-3'

Oligonucleotide 18:

5'-acgactgtagtccaagtaacg-3'

Oligonucleotide 19:
5′-tagcggccgcaagtctgatacgacccggta-3′

Oligonucleotide 20:
5′-gaactagtaacgactgtagtccaagtaacg-3′

As a result, in the case where, as a comparative example, the cDNA fragment derived from the gene E was recovered in the same manner as in Example 1, approximately 80% of the recombinant plasmids subjected to the gene analysis contained the cDNA fragment of interest. In contrast, in the case where the cDNA fragment derived from the gene E was recovered by the method of the present example, approximately 100% of the recombinant plasmids subjected to the gene analysis contained the cDNA fragment of interest.

From these results, it can be found that if the cDNA fragment of interest is recovered from the group of cDNA fragments according to the method of the present example, the cDNA fragment of interest can be recovered much more reliably than the method in Example 1.

In addition, for other parts similar to those of the above-mentioned Example 1, similar effects are obtained.

### [Example 3]

Next, a third example will be described. In the following, the description regarding the same procedures as those of the above-mentioned examples will be omitted or simplified.

Although the cDNA fragments derived from the known genes were recovered and analyzed in Example 1 and Example 2, a cDNA fragment derived from an unknown gene is recovered and analyzed in the present example. When the cDNA fragment derived from the unknown gene is analyzed, there are two possible methods for finding out whether the cDNA fragment which has been recovered is the target cDNA fragment or not.

That is, in one method, when a group of cDNA fragments is subjected to gel electrophoresis with a marker and the length of a cDNA fragment of interest is determined precisely based on an agreement between the length of the cDNA fragment and the length of the marker, and so on, whether the cDNA fragment which has been recovered and analyzed is the target cDNA fragment or not is to be determined from the length thereof.

Specifically, procedures up to a first PCR step were carried out in the same manner as the above-mentioned Examples 1 and 2. Subsequently, the group of amplified cDNA fragments was subjected to acrylamide gel electrophoresis. Of fractionated cDNA fragments, a cDNA fragment with a length of 200 bp (including both adaptor sequences) that matched exactly with the marker having a length of 200 bp was selected as a cDNA fragment of interest to be recovered. Thereafter, the cDNA fragment of interest was recovered in the similar manner as Example 1.

Following the recovery step, a second PCR step was carried out. In the present example, 500 ng of a RecA protein of E. coli with respect to 20 pmol of a primer was further mixed in a PCR reaction solution.

A ligation step and an introduction step were then performed in the same manner as the above-mentioned Examples 1 and 2.

A first primer and a second primer used in the first PCR step are composed of oligonucleotide 21 and oligonucleotide 22, respectively. Similarly, a third primer and a forth primer used in the second PCR step are composed of oligonucleotide 23 and oligonucleotide 24, respectively.

Oligonucleotide 21:
5'-aagtctgatacgacccggtt3'

Oligonucleotide 22:

5′-acgactgtagtccaagtaagt-3′

Oligonucleotide 23:

5'-tagcggccgcaagtctgatacgacccggtt-3'

Oligonucleotide 24:

5′-gaactagtaacgactgtagtccaagtaagt-3′

Next, as described in the above Examples 1 and 2, a recombinant plasmid was extracted from transformed E. coli by a method known in the art to determine a base sequence of the inserted cDNA fragment. From the result, a cDNA fragment of the base sequence having a length of 200 bp was determined to be the cDNA fragment of interest while a cDNA fragment of the base sequence not having a length of 200 bp was determined not to be the cDNA fragment of interest. In addition, as a comparative example, a RecA protein was not added in the second PCR step and a PCR reaction employing only a DNA polymerase (KOD Dash) was carried out while keeping other steps the same as those described above, and the recovery rate of the cDNA fragment of interest was examined.

Further, as another comparative example, a PCR reaction using an SSB (single strand DNA binding protein) protein of E. coli instead of the RecA protein was carried out in the second PCR reaction step while keeping other steps the same as those described above, and the recovery rate of the cDNA fragment of interest was examined.

These results are shown in Table 2.

**[Table 2]**

| | Recovery rate of 200 bp fragment |
|---|---|
| KOD Dash | 8.30% |
| KOD Dash + SSB | < 1% |
| KOD Dash + RecA | 33.30% |

As is evident from Table 2, in the recovery of the cDNA fragment having a length of 200 bp, approximately 33.30% of the recombinant plasmids subjected to the analysis contained the cDNA fragment of interest. In contrast, in the case where the RecA protein was not added in the second PCR step, this recovery rate was decreased to 8.30%. Also, in the case where the SSB protein instead of the RecA protein was added in the second PCR step, this recovery rate was decreased to 1% or less.

From these results, it can be found that the addition of the RecA protein in the second PCR reaction can substantially improve the recovery rate of the cDNA fragment of interest. This may be because, when a PCR reaction is performed with the RecA protein mixed in a reaction solution, the amplification of byproducts (nonspecific PCR products) can be kept low without reducing the yield of the cDNA fragment.

In this way, in the present example, PCR in the second PCR step is carried out by mixing the RecA protein in the reaction solution to amplify the group of cDNA fragments. Therefore, the amplification of byproducts (nonspecific products) can be kept low without reducing the yield of the group of cDNA fragments. That is, since the presence of the above homologous recombinant protein can suppress the occurrence of the primer extension reaction caused by the binding of the primer to nonspecific sites of the cDNA fragment, the amplification of nonspecific PCR products can be suppressed.

In addition, because of high specificity mentioned above, even if the temperature conditions of the primer extension reaction such as an annealing temperature are altered, the group of cDNA fragments can be amplified specifically. That is, a conventional PCR method amplifies not only a group of cDNA fragments but byproducts in a large amount when the temperature of the primer extension reaction such as the annealing temperature is set at a low level. However, in the present example, it is possible to amplify the group of cDNA fragments more specifically.

As a result, the cDNA fragment of interest can be recovered more reliably.

Additionally, since the RecA protein in the range of 1 ng to 1000 ng is mixed for 20 pmol of the primer in the present example, it is possible to amplify a cDNA fragment more efficiently and specifically.

Next, another method in which a cDNA fragment derived from an unknown gene is recovered and analyzed will be described. If the length of a cDNA fragment of interest is not precisely determined even by subjecting the group of cDNA fragments to gel electrophoresis, one set of appropriate primers is prepared on the basis of a base sequence of the cDNA fragment which has been analyzed and recovered, and RT-PCR is carried out to find out whether the cDNA fragment analyzed by RT-PCR is of interest or not.

Specifically, procedures up to a first PCR step were carried out in the same manner as the above-mentioned Examples 1 and 2. Subsequently, the group of amplified cDNA fragments was subjected to acrylamide gel electrophoresis. Of fractionated cDNA fragments, a cDNA fragment having a length of approximately 230 bp (including both adaptor sequences) was selected as a cDNA fragment of interest to be recovered. Thereafter, the cDNA fragment of interest was recovered in the similar manner as Example 1 described above.

Following the recovery step, a second PCR step was carried out.

A ligation step and an introduction step were then performed in the same manner as the above-mentioned Examples 1 and 2.

A first primer and a second primer used in the first PCR step are composed of oligonucleotide 25 and oligonucleotide 26, respectively. Similarly, a third primer and a forth primer used in the second PCR step are composed of oligonucleotide 27 and oligonucleotide 28, respectively.

Oligonucleotide 25:
5′-aagtctgatacgacccgggt-3′

Oligonucleotide 26:
5'-acgactgtagtccaagtaagt-3'

Oligonucleotide 27:
5'-tagcggccgcaagtctgatacgacccgggt-3'

Oligonucleotide 28:

5′-gaactagtaacgactgtagtccaagtaagt-3′

Next, as described in the above-mentioned Examples 1 and 2, a recombinant plasmid was extracted from transformed E. coli by a method known in the art to determine a base sequence of the inserted cDNA fragment. One set of primers was prepared based on the base sequence of a cDNA fragment having a high recovery rate. These primers are composed of oligonucleotide 29 and oligonucleotide 30.

Oligonucleotide 29:
5′-ccagcaacctacaacaaca-3′

Oligonucleotide 30:
5'-caactggtgcatcgccttcat-3'

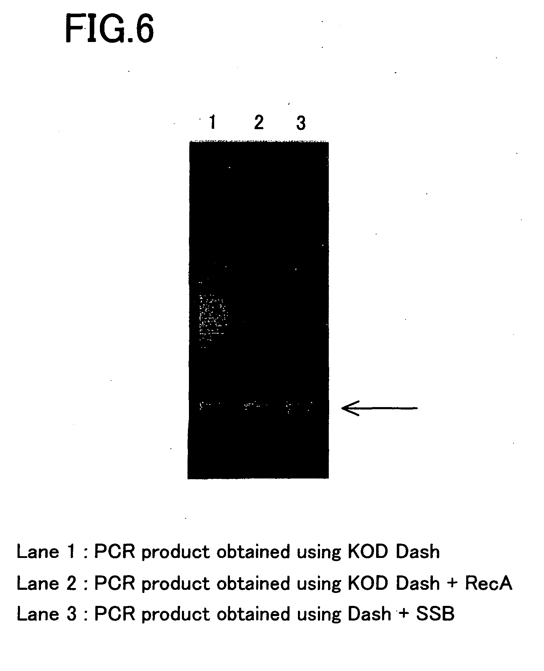

Next, using these primers, a PCR reaction was carried out for the original group of cDNA fragments used as a sample. In addition, as a comparative example, a RecA protein was not added to the sample and a PCR reaction employing only DNA polymerase (KOD Dash) was carried out. Also, as an another comparative example, a PCR reaction using an SSB protein instead of the RecA protein was carried out for the same sample. Subsequently, reaction products thereof were subjected to gel electrophoresis. The results are shown in Fig. 6.

As is evident from Fig. 6, the confirmation of extracted fragments by RT-PCR also gave different results depending on PCR conditions: favorable results and unfavorable results. That is, as shown in Lane 2, when the RecA protein was added to the PCR reaction solution, the cDNA fragment of interest was amplified specifically. In contrast, as shown in Lane 1, when a PCR reaction was carried out using only KOD Dash (DNA polymerase), the whole lane became smeared and the amplification of the cDNA fragment of interest only was not attained. As indicated in Lane 3, when the SSB protein was added in place of the RecA protein, smeared appearance of the lane was improved but an extra band was seen. This may be attributed to the amplification of cDNA fragments other than the cDNA fragment of interest.

From these results, it is found that in the confirmation of the cDNA fragment of interest by RT-PCR, only the cDNA fragment of interest can be amplified specifically by the addition of the RecA protein. Furthermore, the addition of the RecA protein is effective in the confirmation of the recovered cDNA fragments.

Up to this point, the embodiment of the present invention has been described according to examples. However, it is to be understood that the present invention is not intended to be limited to the above examples and various changes may be made therein without departing from the spirit of the present invention.

### [Industrial applicability]

As is evident from the above description, the present invention can provide a method for DNA recovery, in which a cDNA fragment of interest can be recovered more reliably from the group of cDNA fragments in which various kinds of cDNA fragments are present.

## Claims

1. A method for DNA recovery comprising:
a first PCR step for carrying out a PCR reaction on a group of cDNA fragments each having a first adaptor sequence on one end and a second adaptor sequence on the other end, using a first primer having a sequence complementary to the first adaptor sequence and also having a labeling substance and a second primer having a sequence complementary to the second adaptor sequence;
an electrophoretic step for carrying out a gel electrophoresis on the group of cDNA fragments amplified in the first PCR step; and,
on the basis of the results of the electrophoretic step, a recovery step for cutting out a gel portion containing a cDNA fragment of interest from a gel and then recovering the cDNA fragment from the gel.

2. The method for DNA recovery according to Claim 1, further comprising a second PCR step for carrying out a PCR reaction again on the cDNA fragment recovered in the recovery step, using a third primer having a sequence complementary to the first adaptor sequence and a fourth primer having a sequence complementary to the second adaptor sequence.

3. The method for DNA recovery according to Claim 2, wherein in the second PCR step, the PCR is carried out by mixing a homologous recombinant protein containing at least either of a RecA protein or a RecA-altered protein obtained by altering the RecA protein and having functions analogous to those of the RecA protein in a reaction solution.

4. The method for DNA recovery according to any of Claims 1 to 3, further comprising:
a ligation step of forming a recombinant plasmid by ligating the cDNA fragment with a plasmid vector after the recovery step, or after the second PCR step when the second PCR step is provided; and
an introduction step of introducing the recombinant plasmid into E. coli.
